# EUROPEAN PATENT SPECIFICATION

(11) **EP 1 487 402 B1**
(45) Date of publication and mention of the grant of the patent: **18.06.2008**
(21) Application number: 03710567.3
(22) Date of filing: 11.03.2003
(51) Int. Cl.: A61Q 17/04

(54) **COMPOSITION INCLUDING A PIGMENT ASSEMBLY COMPRISING A MICA CORE**
ZUSAMMENSETZUNG MIT EINER EINEN GLIMMERKERN ENTHALTENDEN PIGMENTANORDNUNG
COMPOSITION CONTENANT UN ENSEMBLE PIGMENTAIRE PRESENTANT UN NOYAU EN MICA

(30) Priority: 12.03.2002 SE 0200758
(43) Date of publication of application: 22.12.2004
(73) Proprietor: OVE Karlsson Konsult, 445 36 Bohus (SE)
(72) Inventor: TAMMIK, Rein, S-431 21 Mölndal (SE)
(86) International application number: PCT/SE2003/000413
(87) International publication number: WO 2003/075876

(56) References cited:
- EP-A1- 0 414 049
- EP-A1- 0 998 901
- EP-A2- 0 558 881
- WO-A1-93/11199
- WO-A1-98/01107
- US-A- 5 968 531
- US-A- 6 132 745
- PATENT ABSTRACTS OF JAPAN vol. 199, no. 707 31 July 1997 & JP 09 059 129 A (CATALYSTS & CHEM. IND. CO. LTD.) 04 March 1997
- DATABASE WPI Week 199549, Derwent Publications Ltd., London, GB; Class D21, AN 1995-379946, XP002001246 & JP 7 258 031 A (TOPY KOGYO KK) 09 October 1995
- DATABASE WPI Week 199840, Derwent Publications Ltd., London, GB; Class D21, AN 1998-462752, XP002964904 & JP 10 194 944 A (POLA CHEM. IND. INC.) 28 July 1998
- DATABASE WPI Week 199940, Derwent Publications Ltd., London, GB; Class D21, AN 1999-473983, XP002964905 & JP 11 199 441 A (NOEVIR KK) 27 July 1999
- DATABASE WPI Week 199335, Derwent Publications Ltd., London, GB; Class D21, AN 1993-278179, XP002964906 & JP 5 194 174 A (DAITO KASEI KOGYO KK) 03 August 1993
- DATABASE WPI Week 199309, Derwent Publications Ltd., London, GB; Class D21, AN 1993-071009, XP002964907 & JP 5 017 329 A (TAYCA CORP.) 26 January 1993
- DATABASE WPI Week 199502, Derwent Publications Ltd., London, GB; Class D21, AN 1995-012461, XP002964908 & KR 9 400 999 B1 (PACIFIC IND. CO.) 08 February 1994
- BALSAM M.S. ET AL.: 'Cosmetics, Science and Technology', vol. 1, 1972 XP002964973 second edition, pages 254-256, 264,265

## Description

### Technical field

The present invention relates to a composition including a pigment assembly comprising a mica core for use as an ultraviolet light blocking agent. The use of said pigment assembly for the manufacture of a therapeutic composition in order to prevent bums and skin cancer on the mammalian skin is also described.

### Background of the invention

The ultraviolet rays (UV) in sunlight can be divided into three wavelengths, UVA (320-400 nm), UVB (290-320 nm) and UVC (<290 nm). Exposure to UVB causes redness of the skin. Basal cell carcinoma, squamous cell skin cancer and malignant melanoma are all related to the exposure of the skin to sunlight.

It is known from US-A-6132745; US-A-5968531; JP-A-7258031; JP-A-10194944; JP-A-11199441; EP-A-414049; EP-A-558881 and WO-A-98/01107 that combinations of mica with metal oxides such as titanium dioxide, iron oxide or chromium oxide can be used as UV screens in compositions for the skin.

UVA radiation penetrates deeper into the skin and causes damage to the elastic fibres in the skin. It can give rise to phototoxic reactions in people being treated with certain medication. UVA radiation is also considered to be involved in the development of skin cancer. One of the problems with exposure to UVA is that it does not give rise to the same redness as the exposure to UVB, misleading the person to expose himself to the sunlight longer than he actually can without getting damages to the skin. Both UVA and UVB can cause skin irritation and stimulate the formation of melanine. UVC does not normally reach the surface of the earth due to absorption by the ozone layer. However, in some regions on earth where the ozone layer is thin, UVC radiation do reach the earth. However, with the threat of environmental changes leading to disturbances of the atmosphere composition surrounding the earth, the amount of UVC radiation reaching the earth in the future might be substantial.

Traditional sunscreen products usually contain chemical UV absorbing agents or physical UV filters. The chemical UV absorbing agents can be for instance octyl methoxy cinnamate or benzophenone-3.

The sun protection factor of a conventional sunscreen product is defined as its ability to protect against skin redness caused by the UVB radiation. The factor is primarily a measure of the UVB radiation. It is believed that the sunscreens poor protection against malignant melanoma is an effect of the good protection against the UVB, which expose the skin to large quantities of the UVA that will not be filtered, causing damage during a substantially long period of time.

The number of people getting skin cancer has increased dramatically during the past years. The most common form is basal cell carcinoma comprising more than 15000 new cases per year in Sweden, as well as about 2600 cases of squamous cell carcinoma and about 1600 cases of malignant melanoma. The common feature for all of these is that they are mainly caused by the ultraviolet radiation of the sun.

The sunscreen products utilising chemical filters in the UVA region have shown problems with degradation products and they also give the false indication, that one can stay longer in the sun since no redness is visible on the skin. Physical filters have then been added and these improve the protection as they are more stable and no degradation products occur. However, when reaching wavelengths over 360 nm, the sun screening effect decreases.

In recent years it has been suspected that also radiation having wavelengths exceeding 360 nm and into the area of visible light can cause cancer. No satisfying protection exists for these wavelengths, which can be used in sunscreen compositions.

Different brands of sunscreen products comprising the same sun protection factor have different abilities to protect against UVA, because the estimation of the factor only is measured with reference to the UVB protection. The warning signal i.e., the reddening of the skin caused by UVB is filtered away and people stay out longer in the sun to get damages of the UVA radiation.

Additionally, a thick layer of sunscreen is required to achieve the necessary concentrations of the chemical filters and these thick layers are often uncomfortable for the user. Therefore, the sunscreen is usually applied in a layer having an insufficient thickness to contain the required concentration of the filter in order to render the necessary protection of the skin of the user. This problem is increased with sunscreen products using titanium or zinc oxide.

The chemical filters offer a poor protection at wavelengths exceeding 360 nm and into the area of visible light. In recent years it has been suspected that also wavelengths exceeding 360 and up to above 400 nm can cause cancer. No satisfactory functioning chemical filters exist for these ranges.

The degradation products formed when the chemical filters are consumed upon exposure to sunlight can give rise to damages. There are reports on traces of these compounds found in breast milk and urine after absorption in the skin. Small children usually have sensitive skin and small internal organs to handle and degrade the chemicals absorbed by the skin. For these reasons, the use of sunscreen compositions comprising chemical filters on small children should be avoided.

The physical filters of the prior art, usually represented by titanium dioxide or zinc dioxide are stable and thus fairly reliable. The disadvantage with these physical filters are that these physical filters offer a poor protection at wavelengths exceeding 360 nm. They are usually white or another solid colour which does not look attractive when the user is sunbathing.

There is therefore a great need for new kinds of sunscreens that can offer a reliable protection.

### Summary of the invention

The object of the present invention is to provide a reliable sunscreen composition protecting the mammalian skin upon exposure of a broad interval of UV radiation.

This object has been solved by providing a composition as claimed in claim 1. The use of a pigment assembly comprising a mica core for the manufacture of a therapeutic composition to be applied onto the skin of a person in need thereof for the prevention of skin damages caused by exposure to ultraviolet radiation is also described.

The human skin is protected against skin damages caused by exposure to ultraviolet radiation by applying a composition including a pigment assembly comprising a mica core onto the skin of a person in need thereof.

The compositions according to the invention provide a protection of the mammalian skin against UV radiation in essentially the whole UV spectrum of 220 to 400 nm. The UV filter according to the invention does not-degrade upon exposure and will thus not give rise to harmful compounds upon absorption of the skin. It will provide a time-independent UV filter and is thus reliable.

Additionally, in a method for the estimation of a sun protection factor in a sun screen composition, said sun screen factor is calculated with respect to the amount of UV filter present in said sun screen composition, where said UV filter has the ability to decrease the transmittance of ultraviolet radiation in the entire range of 220-400 nm without being consumed for a predetermined period of time.

The method of determining a sun protection factor has the advantage of being stable for a certain time period as the UV filter does not degrade or consume during use.

There is also provided a method of determine the suitable thickness- of the sunscreen composition that is to be used for skin protection, said method comprising applying a sunscreen composition onto the skin and then visually establish, due to the colour change visible on the skin caused by pigment assemblies in said composition, the thickness of the composition applied onto the skin.

### Brief description of the drawings

The invention will now be closer described by means of the following drawings, where
Fig. 1 shows the relation between reflected light, transmitted light and absorbed light
Fig. 2 shows schematically the skin with and without a layer of the composition according to the invention exposed to sunlight.
Figs. 3-14 show UV transmittance measurements of compositions according to the invention and reference compostions.

### Detailed description of the invention

The pigment assembly according to the invention includes a mica core. Mica is an aluminosilicate with a layer structure. Mica cores of a suitable size is incorporated in a therapeutic and/or cosmetic composition and is intended to be applied on the skin on a mammal in need thereof for the prevention of redness of the skin and/or damages to the skin caused by exposure to ultraviolet radiation.

The pigment assembly comprising the mica core is coated with at least one metal oxide layer. The metal oxide layer is chosen from the group consisting of TiO₂ and/or Fe₂O₃. The pigment assembly may comprise of two or more different layers of said metal oxides onto said mica core or one layer comprising a mixture of two or more metal oxides. The metal oxide layer has a thickness of 40-80 nm.

In another embodiment, said pigment assembly comprising the mica core and said metal oxide layer is further coated with a dye. The dye can be chosen from the group consisting of iron blue and carmine.

The pigment assembly including the metal oxides and optionally the dye as described above is incorporated in a therapeutic and/or cosmetic composition and is intended to be applied on the skin on a mammal in need thereof for the prevention of redness of the skin and/or damages to the skin caused by exposure to ultraviolet radiation. Said ultraviolet radiation has a wavelength of 220 to 400 nm. The cosmetic and/or therapeutic composition is intended for topical use and is in the form of an oil, cream, lotion, or paste. The composition can be used on the skin of a human, but it may also be used on other mammals for UV protection, e.g., on pets such as dogs having less fur and domestic animals such as horses. The composition can exhibit a colour depending on which pigment assembly is contained in the composition. The composition can also have a pearlescent appearance making the skin sparkle due to the presence of the pigment assemblies.

The composition according to the invention is intended to be applied onto the skin of a person in need thereof for the prevention of skin damages caused by exposure to ultraviolet radiation. Said skin damages may be bums, skin cancer such as basal cell carcinoma, squamous cell carcinoma and malignant melanoma. Said composition comprises 2-20 % by weight of the pigment assembly depending on the desired sunscreening effect of said composition. The higher concentrations can be used when almost no transmittance of the UV radiation is desired.

The pigment assembly comprising a mica core is used, for the manufacture of a therapeutic and/or cosmetic composition to be applied onto the skin of a mammal in need thereof for the prevention of skin damage caused by exposure to ultraviolet radiation. Said ultraviolet radiation has a wavelength of 220 to 400 nm. Said pigment assembly is in the form of a mica core coated with at least one metal oxide layer chosen from the group consisting of TiO₂ and/or Fe₂O₃ as described above for the composition itself. Said skin damages may be redness of the skin, skin bums or skin cancer such as basal cell carcinoma, squamous cell carcinoma and malignant melanoma. The composition includes pigment assemblies in amounts of 2-20 % by weight.

For the protection of the human skin against skin damages caused by exposure to ultraviolet radiation the composition including a pigment assembly comprising a mica core is applied onto the skin of a mammal in need thereof. Said ultraviolet radiation has a wavelength of 220 to 400 nm.

The pigment assembly comprises a mica core coated with at least one metal oxide layer, wherein said metal oxide layer(s) is chosen from the group consisting of TiO₂ and/or Fe₂O₃. The metal oxide layer has a thickness of 40-80 nm. Said metal oxide layer can further be coated with a dye. Said skin damages may be bums or skin cancer such as of basal cell carcinoma, squamous cell carcinoma and malignant melanoma.

The composition can be used to protect the skin of a human, but it may also be used on other mammals for UV protection, e.g., on pets such as dogs having less fur and domestic animals such as horses.

The compositions according to the invention provide a protection of the mammalian skin against UV radiation in essentially the whole UV spectrum of 220 to 400 nm. The UV filter according to the invention-does not degrade upon exposure and will thus not give rise to harmful compounds upon absorption of the skin. It will provide a time-independent UV filter and is thus reliable.

Surprisingly, it has been found that the most effective absorption of UV light will take place when the pigment assembly is made from a mica core coated with either titanium dioxide or iron oxide. However, the thickness of the metal oxide coating plays an important role. For an optimum performance it seems essential that the thickness does not exceed 60 nm, or else the ability to absorb and/or reflect the UV light will decrease. However, the compositions including pigment assemblies having thicker metal oxide layers within the claimed range still have an good effect on absorption and/or reflection of the UV light than prior art compositions.

More specifically, when the wavelength is in the region of 220-350 nm, the most efficient absorption and/or reflection will be earned out using the titan dioxide coating, while a mica/iron oxide pigment with a iron oxide content of 44-%, will be more efficient above 360 nm.

When a beam of light falls upon a surface, it may be reflected, absorbed or transmitted through the surface. It may also be a combination between two or three of these. The beam may consist of a single wavelength or a mix of many. The sun radiates light with a wavelength between 220-2500 nm. The shorter the wavelength, the more energy will be carried and therefore, the more dangerous the radiation in this area will be for the human skin.

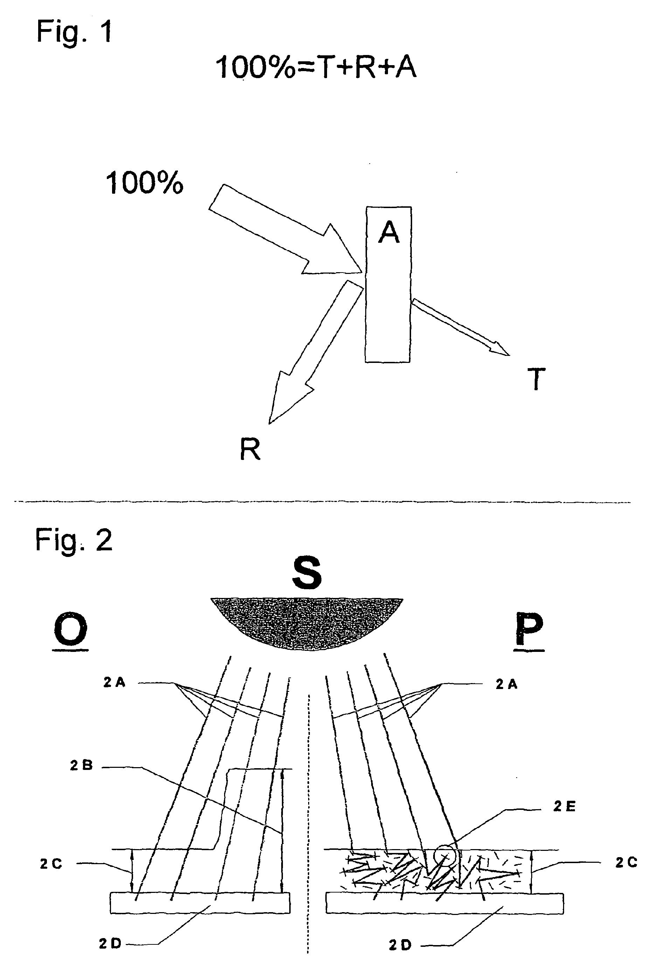

Figure 1 shows the relationship between the incident radiation and absorption A, reflectance R and transmittance T. Sunscreen products can have different amounts of each value A, R and T, but these values always adds up to 100 % of the incident radiation coming from the sun. Depending on the values A, R and T, one will have different results for different sunscreen products regarding its ability to protect the skin against the UV radiation in the sunlight. Obviously, the lesser amount of T, the better the sunscreen product will work.

It is worth mentioning that the wavelength interval of interest regarding the invention is the UV area. Therefore it is important that the value of T is low only up to wavelength 400 nm. Above this value, it is harmless to increase the T-value.

It is not totally evident why the compositions according to the invention are so efficient in prevention the skin from UV radiation. One possible theory is based on reflection. A light beam consists of several photons. Each photon has an energy content being proportional to its wavelength. When a photon having a very short wavelength hits the skin being coated with the composition according to the invention, the photon will bounce due to reflection between the inventive pigment assemblies several times on its way through the composition layer. At each reflection it would lose some of its energy causing an increase of its wavelength. After certain energy losses after a number of reflections, the wavelength of said photon will have a value being less harmful to the skin.

Figure 2 shows the difference between the ways a common sunscreen product using chemical filters and the invention using the composition comprising pigment assemblies according to the invention will work. On the left hand side of the figure marked "O", it is shown how the sunray travels through an ordinary sunscreen product. 2A refers to incident sunrays from the sun S and 2B refers to the thickness one must apply in order to receive a sufficient protection against the UV-radiation. 2C refers to the thickness of the sunscreen product layer that people normally use when sunbathing, meaning that the thickness of the layer is not sufficient to protect the skin from UV-radiation. 2D represents the skin surface.

On the right hand of the figure marked "P", it is shown how the composition according to the invention works. Each time the ray hits a pigment assembly, it will be absorbed to some extent, and it will lose some of its energy via reflection, and as a result, therefore change its wavelength towards a higher value. The remaining amount of radiation then becomes less harmful to the skin. In the figure 2A refers to the incident sunrays from the sun S. 2C refers to the thickness of the sunscreen product layer that people normally use when sunbathing. In this case, using the invention, the layer is sufficient to protect the skin from UV-radiation. 2D represents the skin surface. 2E shows how the sunray will reflect on the surface of a pigment assembly and travel on to the next pigment assembly.

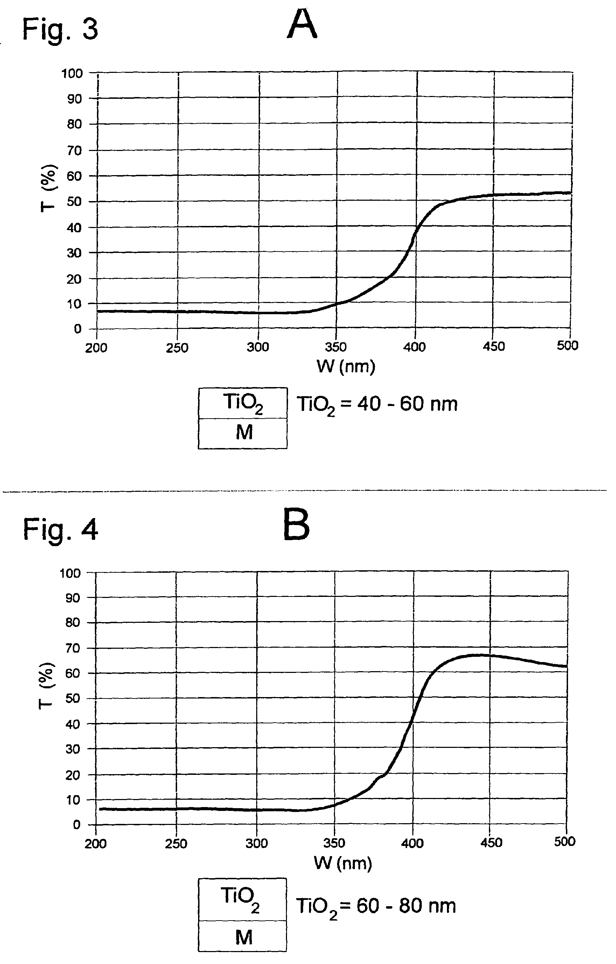

Another reason of the success of the compositions according to the invention might be based on absorption in combination with reflection. When sunlight hits a pigment assembly according to the compositions A, B or E (see figs. 3, 4 and-7), the particular design of the assembly will efficiently absorb a main part of all wavelengths between 220 and 500 nm. These pigment assemblies apparently have the ability to absorb more but reflect and transmit substantially less than other pigment, to render the efficient protection against the harmful UVA, UVB and UVC radiation.

The sun protection factor of a conventional sunscreen product is defined as its ability to protect against skin redness caused by the UVB radiation. The factor does not take into consideration whether the UVA radiation affects the situation or not. The definition of the term "sun screen factor" relates to the current technology for protection against UV-radiation. Using the present invention this term might be redefined to comprise a considerably more relevant meaning. The inventor of the present invention is of the opinion that there is a discrepancy in the use of the term "sun protection factor". The term is a measure of the protection offered from a sunscreen product against the blushing reaction of the skin caused primarily by the UVB radiation. Since all chemical-filters are degraded in the course of time of sun exposure, this leads to a time related change. A certain protection at a given time thus decreases after a certain period of time due to this consumption of the chemical filter. This decreasing of sun protection ability leads to that people can expose themselves to sun longer than the skin can withstand and therefore causing damages to the skin.

To estimate a sun protection factor in a sun screen composition, said sun screen factor is calculated with respect to the amount of UV filter present in said sun screen composition, where said UV filter has the ability to decrease the transmittance of ultraviolet radiation in the entire range of 220-400 nm without being consumed for a predetermined period of time.

The method of determining a sun protection factor has the advantage of being stable for a certain time period as the UV filter does not degrade or consume during use.

There is also provided a method of determine the suitable thickness of the sunscreen composition that is to be used for skin protection, said method comprising applying a sunscreen composition onto the skin and then visually establish, due to the colour change visible on the skin caused by pigment assemblies in said composition, the relevant thickness of the composition applied onto the skin. According to the method, the user of the compositions according to the invention will spread out the composition on the skin. The coloured composition will exhibit a colour on the skin when applied in a thick layer, the colour will disappear when the composition is further applied on a larger surface on the skin leading to a thinner layer of said composition. This colour change indicated for the user of the sun screening composition that a layer that is thick enough to provide a good protection of the skin against the sun has been applied on the skin.

### Experimental section

The invention will now be closer illustrated by means of the following non-limiting examples.

### Preparation of compositions

Compositions including pigment assemblies according to the invention and one reference sample were prepared using conventional method.

| **Composition A** (shown in figure 3) | % w/w |
|---|---|
| Pigment assembly, Iriodine 111 (Merck KgaA - Sparte Pigmente) | 20 |
| White mineral oil, such as BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, such as Vanessence SA Tea-Tree (Parfusale AB) | 0.5 |

| **Composition B** (shown in figure 4) | % w/w |
|---|---|
| Pigment assembly, Iriodine 201 (Merck KgaA - Sparte Pigmente) | 20 |
| White mineral oil, BP Enerpar M 002 (Chematex) | 64.5 |
| Viscosity adjustment, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition C** (shown in figure 5) | % w/w |
|---|---|
| Pigment assembly, Iriodine 223 (Merck KgaA - Sparte Pigmente) | 20 |
| White mineral oil, BP Enerpar M 002 (Chematex) | 64.5. |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition D** (shown in figure 6) | % w/w |
|---|---|
| Pigment assembly, Iriodine 326 (Merck KgaA - Sparte Pigmente) | 20 |
| White mineral oil, BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition E** (shown in figure 7) | % w/w |
|---|---|
| Pigment assembly, Iriodine 520 (Merck KgaA - Sparte Pigmente) | 20 |
| White mineral oil, BP Enerpar M 002 (Chematex) | 64.5 |
| Viscosity adjustment, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition F** (shown in figure 8) | % w/w |
|---|---|
| Pigment assembly, Iriodine 9612 (Merck KgaA - Sparte Pigmente) | 20 |
| White mineral oil, BP Enerpar M 002 (Chematex) | 64.5 |
| Viscosity adjustment, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition G** (shown in figure 9) | % w/w |
|---|---|
| Pigment assembly, Iriodine 527 (Merck KgaA - Sparte Pigmente) | 20 |
| White mineral oil, BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition H** (shown in figure 10) | % w/w |
|---|---|
| Pigment assembly, Iriodine 323 (Merck KgaA - Sparte Pigmente) | 20 |
| White mineral oil, BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition J** (shown in figure 11) | % w/w |
|---|---|
| Pigment assembly, Iriodine 9440 (Merck KgaA - Sparte Pigmente) | 20 |
| White mineral oil, BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition K** (shown in figure 12) | % w/w |
|---|---|
| Pigment assembly, Iriodine 9444 (Merck KgaA - Sparte Pigmente) | 20 |
| White mineral oil, BP Enerpar M 002 (Chematex) | 64.5 |
| Viscosity adjustment agent, such as Wacker HDK 20 (Wacker- Kemi AB) | 6 |
| Conditioning agent, such as Estol 1514 (Uniqema) | 4 |
| Conditioning agent, such as Estol 3603 (Uniqema) | 5 |
| Parfume, Vanessence SA Tea-Tree (Parfusale AB) | 0.5 |

| **Composition OMC** (shown in figure 13) | % w/w |
|---|---|
| Octyl methoxycinnamate, Parsol MCX (Parfusale AB) | 6 |
| White mineral oil, BP Enerpar M 002 | 78.5 |
| Viscosity adjustment agent, such as Wacker HDK 20 | 6 |
| Conditioning agent, such as Estol 1514 | 4 |
| Conditioning agent, such as Estol 3603 | 5 |
| Parfume, such as Vanessence SA Tea-Tree | 0.5 |

| **Composition Aa** (shown in figure 14) | % w/w |
|---|---|
| Pigment assembly, Iriodine 111 (Merck KgaA - Sparte Pigmente) | 5 |
| White mineral oil, BP Enerpar M 002 (Chematex) | 79.5 |
| Viscosity adjustment agent, such as Wacker HDK 20 (Wacker- Kemi AB) | 6 |
| Conditioning agent, Estol 1514 (Uniqema) | 4 |
| Conditioning agent, Estol 3603 (Uniqema) | 5 |
| Parfume, Vanessence SA Tea-Tree (Parfusale AB) | 0.5 |

### Test of compositions

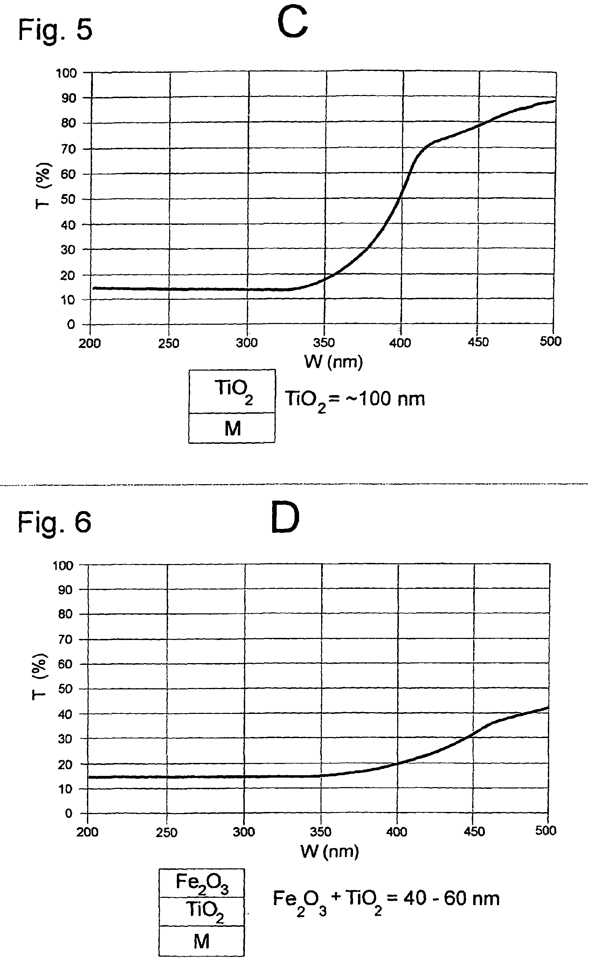

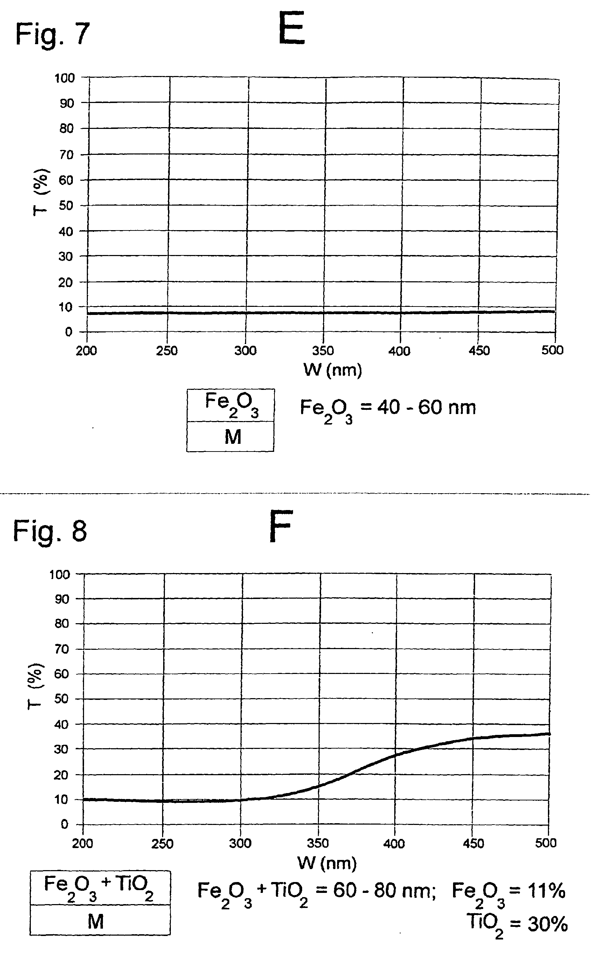

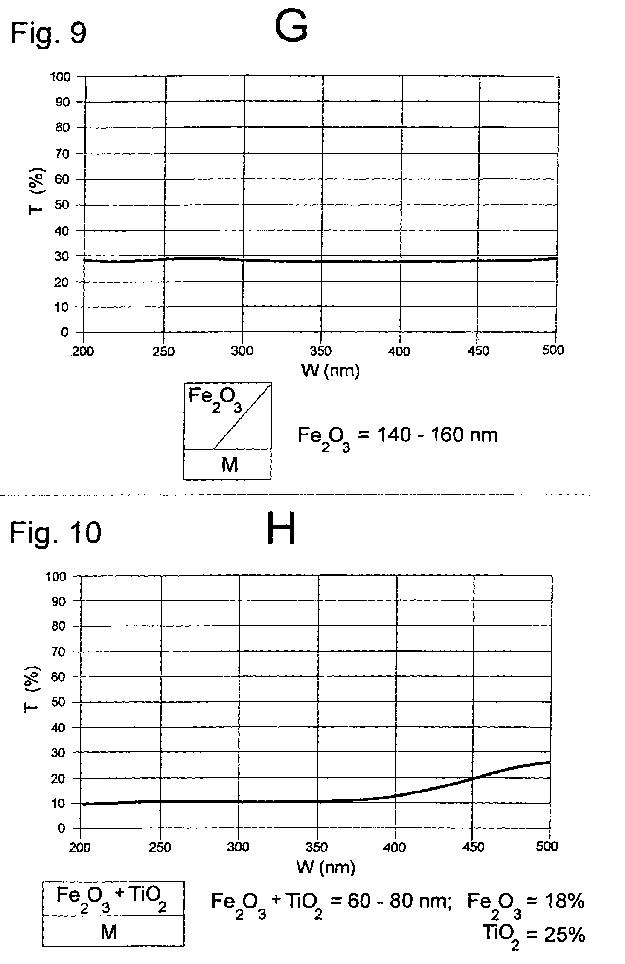

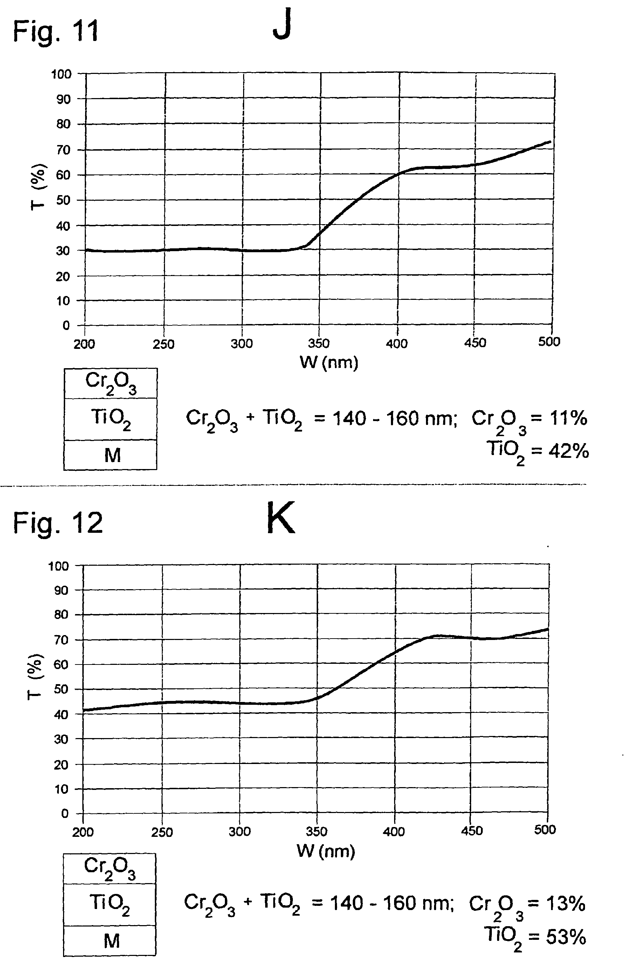

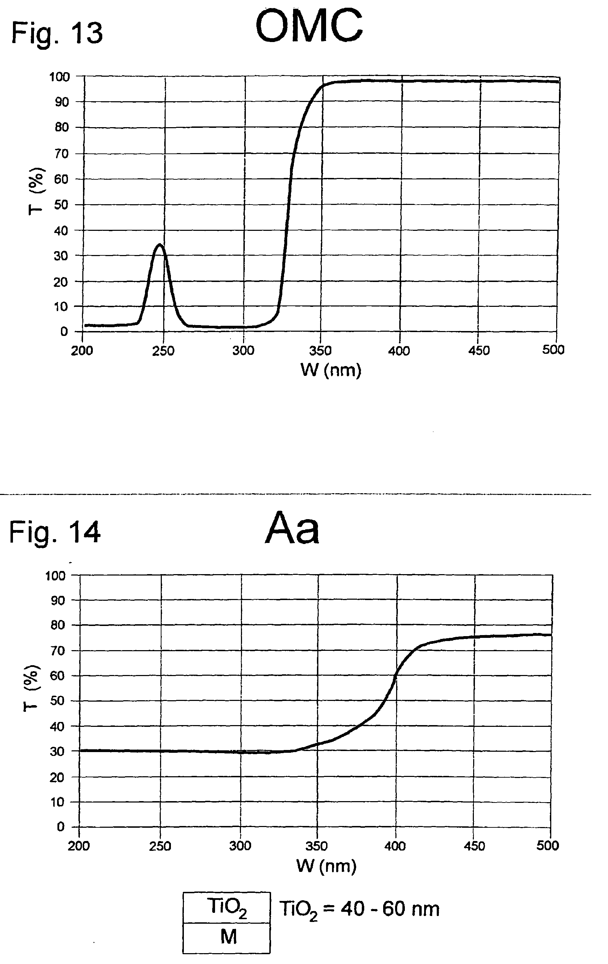

The compositions according to the invention were tested for transmittance of light having a wavelength from about 220-500 nm in a UV-VIS-NIR spectrophotometer (Perkin-Elmer Lambda 9). The transmittance was estimated as the intensity of incident radiant flux divided with the intensity of transmitted radiant flux. Reference is given to Möller et al., J. Appl. Pol. Sci., Vol. 61, 1149-1162 (1996) for a more detailed description of the method and the equipment used. The results of the measurements are shown in Figs. 3-14. Fig. 13 shows a reference spectrum of a conventionally used chemical UV absorbing agent, namely OMC (octyl methoxy cinnamate).

In the figures 3-14, the X-axis represented by W (nm) is the wavelength of the incident radiation and the Y-axis, represented by T (%), is the transmittance expressed in % of the incident radiation.

The different figures 3-12 shows the different effects that will be obtained using differently constructed pigment assemblies. A drawing of the pigment assembly used in each composition is shown underneath each graph, denoting the thickness of the metal oxide layer covering the mica core, denoted M. The metal oxide layers are denoted using their chemical formula, wherein TiO₂ is titanium dioxide, Fe₂O₃ is iron oxide, and Cr₂O₃ is chrome oxide. The figures all show that the compositions A-K, and Aa are more or less effective in the above tested wavelength interval from about 220 to 500 nm indicating their potential use in sunscreening products.

Compositions A, B, D, E, F, H and Aa are according to the invention.

The figure 14 shows that the concentration of the pearl pigment in the composition is directly proportional to the ability to absorb and reflect the UV-radiation. The pigment construction in figure 14 called Aa is exactly the same as in figure 3 called A. The only difference is the pigment concentration, which is 5 % (weight %) in Aa compared with 20 % (weight %) in A.

## Claims

1. A cosmetic and/or therapeutic composition for topical use in the form of an oil, cream, lotion or paste comprising 2-20% by weight of a pigment assembly in the form of a mica core coated with at least one metal oxide layer chosen from the group consisting of TiO2 and/or Fe203 and wherein the thickness of the metal oxide layer is 40-80 nm.

2. Composition according to claim 1, wherein said metal oxide layer consist of Fe₂O₃ and has a thickness of 40-60 nm.

3. Composition according to claim 1 or 2, wherein said metal oxide layer is coated with a dye.

4. Composition according to claim 3, wherein said dye is chosen from the group consisting of iron blue and carmine.

5. Composition according to any of the claims 1-4, wherein said composition has a pearlescent appearance.

6. A method to determine the suitable thickness of the composition according to claims 4 and 5 that is to be used to provide desired skin protection against ultraviolet radiation, comprising:
a) that the composition is applied onto the skin;
b) that the composition is spread out to a certain thickness on the skin; and
c) that one visually establishes the thickness that has been applied on the skin and the sun protection that then has been obtained by observing the visible colour change on the skin, caused by the pigment assemblies in said composition.

## Patentansprüche

1. Kosmetische und/oder therapeutische Zubereitung für die topische Verwendung in Form eines Öls, einer Creme, einer Lotion oder Paste umfassend 2-20 Gew.-% einer Pigmentanordnung in Form eines Glimmerkerns, beschichtet mit mindestens einem Metalloxidüberzug, ausgewählt aus der Gruppe von TiO₂ und/oder Fe₂O₃ wobei die Dicke des Metalloxidüberzugs 40 - 80 nm beträgt,

2. Zubereitung gemäß Anspruch 1, wobei der Metalloxidüberzug aus Fe₂O₃ besteht und eine Dicke von 40 - 60 nm hat.

3. Zubereitung gemäß Anspruch 1 oder 2, wobei der Metalloxidüberzug mit einem Farbstoff beschichtet: ist.

4. Zubereitung gemäß Anspruch 3, wobei der Farbstoff aus der Gruppe von Eisenblau und Karmin ausgewählt wird.

5. Zubereitung gemäß einem der Ansprüche 1-4, wobei die Zubereitung einen perlmuttartigen, Glanz hat.

6. Verfahren zur Bestimmung der geeigneten Dicke der Zubereitung gemäß Ansprüchen 4 und 5, welche für den gewünschten Hautschutz gegen Ultraviolette Strahlen verwendet wird, umfassend:
a) dass die Zubereitung auf der Haut aufgetragen wird;
b) dass die Zubereitung mit einer bestimmen Dicke auf der Haut verteilt wird; und
c) dass man visuell die Dicke, welche auf der Haut aufgetragen wurde, und der Sonnenschutz, der dann erhalten wird, durch die Wahrnehmung der sichtbaren Farbveränderung auf der Haut, bedingt durch die Pigmentanordnung der Zubereitung, ermittelt.

## Revendications

1. Composition cosmétique et/ou thérapeutique pour une utilisation topique dans la forme d'une huile, d'une crème, d'une lotion ou d'une pâte comprenant 2-20% en poids d'un assemblage de pigment dans la forme d"un noyau de mica revêtu avec au moins une couche d'oxyde métallique choisi parmi TiO₂ et/ou Fe₂O₃ et dans laquelle l'épaisseur de la couche d'oxyde métallique est de 40-80nm.

2. Composition selon la revendication 1, dans laquelle ladite couche d'oxyde métallique est constituée de Fe₂O₃ et présente une épaisseur de 40-60 nm.

3. Composition selon la revendication 1 ou 2, dans laquelle ladite couche d'oxyde métallique est revêtue d'un colorant.

4. Composition selon la revendication 3, dans laquelle ledit colorant est choisi parmi le bleu de fer et le carmin.

5. Composition selon l'une quelconque des revendications 1-4, dans laquelle ladite composition présente une apparence de perle.

6. Procédé pour déterminer l'épaisseur appropriée de la composition selon les revendications 4 et 5 qui est à utiliser pour fournir une protection souhaitée de la peau contre un rayonnement ultraviolet consistant en ce que :
a) la composition est appliquée sur la peau;
b) la composition est étalée sur une certaine épaisseur sur la peau ; et
c) on établit visuellement l'épaisseur qui a été appliquée sur la peau et la protection solaire qui a été obtenue en observant la modification visible de la couleur sur la peau, occasionnée par les assemblages de pigment dans ladite composition.
